# EUROPEAN PATENT APPLICATION

(11) **EP 4 516 253 A1**
(43) Date of publication of application: **05.03.2025**
(21) Application number: 24178617.7
(22) Date of filing: 28.05.2024
(51) Int. Cl.: A61B 34/10

(54) **TEMPOROMANDIBULAR JOINT PROSTHESIS IMPLANTATION EQUIPMENT AND STORAGE MEDIUM**

(30) Priority: 31.08.2023 CN 202311121908
(71) Applicant: Gaofeng Medical Equipment (Wuxi) Co., Ltd, Huishan Economic Development Zone Wuxi, Jiangsu 214000 (CN)
(72) Inventor: BAO, Yaxing, Wuxi, 214000 (CN); GAO, Jun, Wuxi, 214000 (CN); GAO, Xingrong, Wuxi, 214000 (CN)
(74) Representative: Chung, Hoi Kan

(57) **Abstract**

The present invention relates to the field of biomedical technology. The description discloses a temporomandibular joint prosthesis implantation method, device, equipment and storage medium, wherein the method comprises: conducting total image scanning on a target temporomandibular joint region to obtain scanned image data; generating a three-dimensional solid mathematical model based on the scanned image data; determining an implantation cutting line and a preset temporomandibular joint prosthesis according to the three-dimensional solid mathematical model. According to the present invention, the three-dimensional solid mathematical model is generated based on the scanned image data obtained by conducting total image scanning on the target temporomandibular joint region, and the implantation cutting line and the preset temporomandibular joint prosthesis that match the target temporomandibular joint region are determined according to the three-dimensional solid mathematical model, so that the temporomandibular joint prosthesis can be implanted based on the implantation cutting line and the preset temporomandibular joint prosthesis, which ensures the implantation accuracy of the temporomandibular joint prosthesis.

## Description

### Technical field

The present invention relates to the field of biomedical technology, in particular to a temporomandibular joint prosthesis implantation method, device, equipment and storage medium.

### Background art

TMJ, also known as temporomandibular joint or mandibular joint, is composed of caput mandibulae, fossa mandibularis ossis temporalis and tuberculum articulare, and the left and right are combined into one combined joint. Temporomandibular joint is mainly responsible for mouth opening and mouth closing and chewing, so for users with poor temporomandibular joint movement, the joint movement of users can be improved by implanting temporomandibular joint prosthesis, so that users are able to normally open and close their mouths and chew.

The traditional temporomandibular joint prosthesis implantation method usually relies on the artificial experience of the operators, but there are too many uncontrollable factors (such as limited experience and wrong operation) of this method, which makes the implantation accuracy of the prosthesis unable to be ensured in the current temporomandibular joint prosthesis implantation process, which results in the problem that the users still have poor temporomandibular joint movement after prosthesis implantation. Therefore, there is an urgent need for a temporomandibular joint prosthesis implantation method that can improve the implantation accuracy.

The foregoing contents are only for assisting in understanding the technical solutions of the present invention, but do not represent an admission that the foregoing contents are prior art.

### Summary of the invention

The main purpose of the present invention is to provide a temporomandibular joint prosthesis implantation method, device, equipment and storage medium, which aims at solving the technical problem that the implantation accuracy of the temporomandibular joint prosthesis cannot be ensured in the prior art.

In order to achieve the foregoing purpose, the present invention provides a temporomandibular joint prosthesis implantation method, wherein the method comprises the following steps:
Conducting total image scanning on a target temporomandibular joint region to obtain scanned image data;
Generating a three-dimensional solid mathematical model based on the scanned image data, wherein the three-dimensional solid mathematical model comprises a temporomandibular joint region to be implanted;
Determining an implantation cutting line and a preset temporomandibular joint prosthesis according to the three-dimensional solid mathematical model;
Implanting a temporomandibular joint prosthesis in the target temporomandibular joint region based on the implantation cutting line and the preset temporomandibular joint prosthesis;
Alternatively, the scanned image data comprises first scanned image data and second scanned image data, and the step of conducting total image scanning on the target temporomandibular joint region to obtain the scanned image data comprises:
   Adjusting an oral state corresponding to the target temporomandibular joint region to an occlusal state, and conducting total image scanning on the temporomandibular joint region in the occlusal state to obtain the first scanned image data;
   Adjusting the oral state corresponding to the target temporomandibular joint region to a maximum mouth opening state, and conducting total image scanning on the temporomandibular joint region in the maximum mouth opening state to obtain the second scanned image data;
   Alternatively, the three-dimensional solid mathematical model comprises a first mathematical model and a second mathematical model, wherein the step of generating the three-dimensional solid mathematical model based on the scanned image data comprises:
      Conducting data processing on the scanned image data to obtain processed first scanned image data and processed second scanned image data;
      Generating the first mathematical model based on the processed first scanned image data;
      Generating the second mathematical model based on the processed second scanned image data.

Alternatively, the step of determining the implantation cutting line and the preset temporomandibular joint prosthesis according to the three-dimensional solid mathematical model comprises:
Locating the temporomandibular joint region to be implanted corresponding to the target temporomandibular joint region from the first mathematical model, and determining the implantation cutting line based on the temporomandibular joint region to be implanted, wherein the implantation cutting line comprises an upper cutting line and a lower cutting line;
Determining the preset temporomandibular joint prosthesis according to the first mathematical model and the second mathematical model, wherein the preset temporomandibular joint prosthesis comprises a joint fossa prosthesis, a joint head prosthesis, a joint stem prosthesis and a guide plate for surgical implantation;
Alternatively, the step of determining the preset temporomandibular joint prosthesis according to the first mathematical model and the second mathematical model comprises:
   Establishing a Cartesian right-hand coordinate system according to the first mathematical model and the second mathematical model;
   Constructing the preset temporomandibular joint prosthesis adapted to the temporomandibular joint region in the Cartesian right-hand coordinate system, wherein in the preset temporomandibular joint prosthesis, the joint fossa prosthesis and the joint stem prosthesis comprise a plurality of fastening screw holes thereon, and the guide plate for surgical implantation comprises a plurality of fastening screw holes and a plurality of positioning screw holes thereon.

Alternatively, the step of implanting a temporomandibular joint prosthesis in the target temporomandibular joint region based on the implantation cutting line and the preset temporomandibular joint prosthesis comprises:
Cutting the temporomandibular joint region to be implanted based on the implantation cutting line, and implanting the preset temporomandibular joint prosthesis into the target temporomandibular joint region by using screws with the fastening screw holes as the fastening reference and the positioning screw holes as the positioning reference after completing cutting.

Alternatively, after the step of implanting a temporomandibular joint prosthesis in the target temporomandibular joint region based on the implantation cutting line and the preset temporomandibular joint prosthesis, it further comprises:
When the preset temporomandibular joint prosthesis is implanted into the target temporomandibular joint region, removing the guide plate for surgical implantation and stitching the wound surface corresponding to the target temporomandibular joint region.

In addition, in order to achieve the foregoing purpose, the present invention further provides a temporomandibular joint prosthesis implantation device, which comprises:
An image scanning module, configured to conduct total image scanning on a target temporomandibular joint region to obtain scanned image data;
A model generation module, configured to generate a three-dimensional solid mathematical model based on the scanned image data, wherein the three-dimensional solid mathematical model comprises a temporomandibular joint region to be implanted;
A data acquisition module, configured to determine an implantation cutting line and a preset temporomandibular joint prosthesis according to the three-dimensional solid mathematical model;
A prosthesis implantation module, configured to implant a temporomandibular joint prosthesis in the target temporomandibular joint region based on the implantation cutting line and the preset temporomandibular joint prosthesis.

In addition, in order to achieve the foregoing purpose, the present invention further provides temporomandibular joint prosthesis implantation equipment, which comprises a memory, a processor, and a temporomandibular joint prosthesis implantation program that is stored in the memory and can run on the processor, wherein the temporomandibular joint prosthesis implantation program is configured to carry out the steps of the temporomandibular joint prosthesis implantation method as described above.

In addition, in order to achieve the foregoing purpose, the present invention further provides a storage medium, wherein the temporomandibular joint prosthesis implantation program is stored on the storage medium, and when the temporomandibular joint prosthesis implantation program is executed by the processor, the steps of the temporomandibular joint prosthesis implantation method described above are carried out.

According to the present invention, total image scanning on a target temporomandibular joint region is conducted to obtain scanned image data; a three-dimensional solid mathematical model is generated based on the scanned image data, and the three-dimensional solid mathematical model comprises a temporomandibular joint region to be implanted; an implantation cutting line and a preset temporomandibular joint prosthesis are determined according to the three-dimensional solid mathematical model; and a temporomandibular joint prosthesis is implanted into the target temporomandibular joint region based on the implantation cutting line and the preset temporomandibular joint prosthesis. Compared with the prior art that the temporomandibular joint prosthesis implantation is carried out through the manual experience of workers, since according to the foregoing method of the present invention, the three-dimensional solid mathematical model is generated based on the scanned image data obtained by conducting total image scanning on the target temporomandibular joint region, and the implantation cutting line and the preset temporomandibular joint prosthesis that match the target temporomandibular joint region are determined according to the three-dimensional solid mathematical model, so that the temporomandibular joint prosthesis is implanted based on the implantation cutting line and the preset temporomandibular joint prosthesis, which avoids the technical problem in the prior art that the users still have poor temporomandibular joint movement after prosthesis implantation due to uncontrollable factors such as inexperience and incorrect operation of workers, and the like, which further ensures the implantation accuracy of the temporomandibular joint prosthesis.

### Description of drawings

Figure 1 is a structural diagram of temporomandibular joint prosthesis implantation equipment in a hardware runtime environment involved in an embodiment of the present invention;
Figure 2 is a flow chart of a first embodiment of a temporomandibular joint prosthesis implantation method of the present invention;
Figure 3 is a flow chart of a second embodiment of the temporomandibular joint prosthesis implantation method of the present invention;
Figure 4 is a flow chart of a third embodiment of the temporomandibular joint prosthesis implantation method of the present invention;
Figure 5 is a schematic diagram of a preset temporomandibular joint prosthesis in the temporomandibular joint prosthesis implantation method of the present invention; and
Figure 6 is a structural block diagram of a first embodiment of a temporomandibular joint prosthesis implantation device of the present invention.

The realization, functional characteristics and advantages of the present invention will be further described with reference to the attached drawings in combination with embodiments.

### Embodiments

It should be understood that the specific embodiments described herein are only for explaining the present invention, but are not for limiting the present invention.

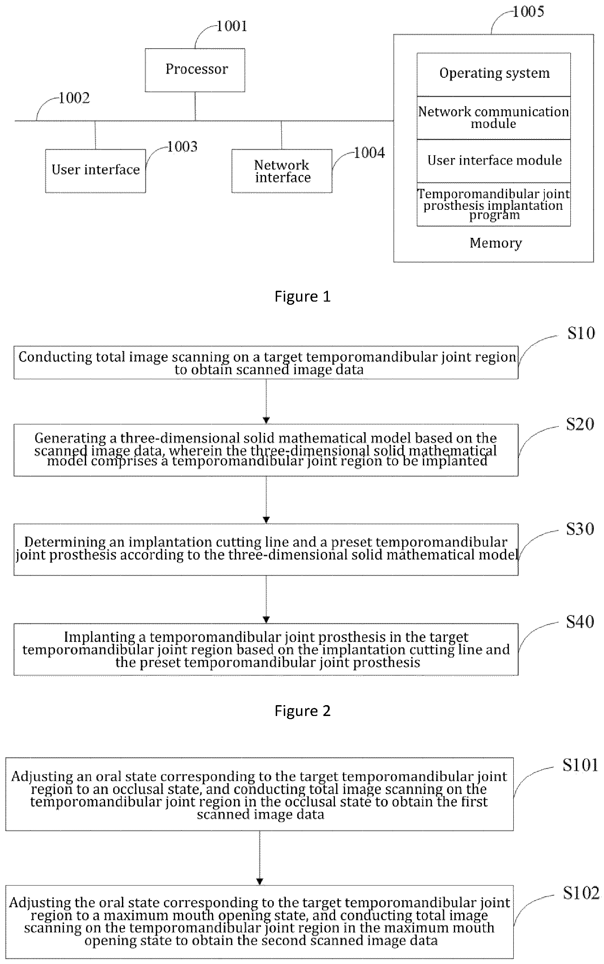

With reference to Figure 1, Figure 1 is a structural diagram of temporomandibular joint prosthesis implantation equipment in a hardware runtime environment involved in an embodiment of the present invention;
As shown in Figure 1, the temporomandibular joint prosthesis implantation equipment may comprise a processor 1001, such as a Central Processing Unit (CPU), a communication bus 1002, a user interface 1003, a network interface 1004, and a memory 1005. Wherein, the communication bus 1002 is for realizing connection communication between these components. The user interface 1003 may comprise a Display and an input unit such as a Keyboard, and the optional user interface 1003 may comprise a standard wired interface and a wireless interface. The network interface 1004 may optionally comprise a standard wired interface and a wireless interface (such as a Wireless-Fidelity (Wi-Fi) interface). The memory 1005 may be a high-speed Random Access Memory (RAM) or a stable Non-Volatile Memory (NVM), such as a disk memory. The memory 1005 may alternatively be a storage device independent of the aforementioned processor 1001.

It can be understood by those skilled in the art that the structure shown in Figure 1 does not constitute a limitation on the temporomandibular joint prosthesis implantation equipment, and may comprise more or less components than shown, or combine some components, or have different layouts of components.

As shown in Figure 1, the memory 1005 as a storage medium may comprise an operating system, a network communication module, a user interface module and a temporomandibular joint prosthesis implantation program.

In the temporomandibular joint prosthesis implantation equipment as shown in Figure 1, the network interface 1004 is mainly configured for data communication with the network server; the user interface 1003 is mainly configured for data interaction with users; the processor 1001 and the memory 1005 in the temporomandibular joint prosthesis implantation equipment of the present invention can be configured in the temporomandibular joint prosthesis implantation equipment, and the temporomandibular joint prosthesis implantation equipment calls the temporomandibular joint prosthesis implantation program stored in the memory 1005 through the processor 1001 and executes the temporomandibular joint prosthesis implantation method provided by the embodiment of the present invention.

The embodiment of the present invention provides a temporomandibular joint prosthesis implantation method, with reference to Figure 2, Figure 2 is a flow chart of a first embodiment of a temporomandibular joint prosthesis implantation method of the present invention.

In the present embodiment, the temporomandibular joint prosthesis implantation method comprises the following steps:
Step S10, conducting total image scanning on a target temporomandibular joint region to obtain scanned image data.

It should be noted that the execution subject of the method in the present embodiment can be a computing service device with data processing, network communication and program running functions, such as a mobile phone, a tablet computer, a personal computer, etc., and can also be other electronic devices that can carry out the same or similar functions, and the embodiment is not limited thereto. Herein, embodiments of the temporomandibular joint prosthesis implantation method of the present invention will be described by taking the temporomandibular joint prosthesis implantation equipment (hereinafter referred to as the implantation equipment) as an example.

It can be understood that the foregoing scanned image data can be two-dimensional image data, such as image data in DICOM (digital imaging and communications in medicine) format, etc., and the embodiment is not limited thereto.

In a specific implementation, CT (Computed Tomography) imaging technology can be used to conduct total image scanning on the target temporomandibular joint region to obtain the scanned image data. Wherein, CT imaging technology can measure the human body with highly sensitive instruments according to the differences of X-ray absorption and transmittance of different tissues in the target temporomandibular joint region, and then input the data obtained from the measurement into the implantation equipment, and after the implantation equipment processes the data, the image data of the inspected part in the target temporomandibular joint region can be taken, which is the scanned image data.

Step 520, generating a three-dimensional solid mathematical model based on the scanned image data, wherein the three-dimensional solid mathematical model comprises a temporomandibular joint region to be implanted.

It should be noted that the three-dimensional solid mathematical model can be a three-dimensional representation of the scanned image data.

It should be understood that the temporomandibular joint region to be implanted can be the region that needs to be implanted with the temporomandibular joint prosthesis in the three-dimensional solid mathematical model (such as the lesion region of the temporomandibular joint).

In a specific implementation, the scanned image data can be converted into the three-dimensional solid mathematical model by SSD (Shaded Surface Display) technology, namely, the scanned image data is calculated and processed according to the surface mathematical model, and the adjacent pixels exceeding the preset signal threshold are connected to form an image of light and dark areas, so as to display the three-dimensional relationship in complex and overlapping structures and the surface morphology of related structures, and to obtain the three-dimensional solid mathematical model.

In addition, VR (Volume Rendering) technology can also be adopted to convert the scanned image data into the three-dimensional solid mathematical model, namely, the CT values of all voxels in the scanned image data are set to different transparency, from completely opaque to completely transparent, and at the same time, a three-dimensional image is displayed with different gray scales or false colors by virtual lighting effect, and the three-dimensional image is the three-dimensional solid mathematical model. The foregoing discussion is only for illustrating the generation process of the three-dimensional solid mathematical model rather than limiting it. Other methods that can convert the scanned image data into the three-dimensional solid mathematical model are also applicable to the embodiment, which will not be repeated herein.

Step 530, determining an implantation cutting line and a preset temporomandibular joint prosthesis according to the three-dimensional solid mathematical model;
It should be noted that the implantation cutting line can be a cutting line of the lesion region of temporomandibular joint.

It should be understood that the preset temporomandibular joint prosthesis can be a prosthesis that needs to be implanted into the temporomandibular joint region to replace the cut lesion region of the temporomandibular joint in the present embodiment.

In a specific implementation, the region can be divided in the three-dimensional solid mathematical model, so as to determine the implantation cutting line; solid simulation can be carried out in the foregoing three-dimensional solid mathematical model, so as to determine the preset temporomandibular joint prosthesis that matches the real user.

Step S40, implanting a temporomandibular joint prosthesis in the target temporomandibular joint region based on the implantation cutting line and the preset temporomandibular joint prosthesis;
In a specific implementation, the target temporomandibular joint region can be cut according to the implantation cutting line, and the preset temporomandibular joint prosthesis can be used to replace the cut part in the target temporomandibular joint region to carry out the implantation of the temporomandibular joint prosthesis.

According to the present embodiment, total image scanning on a target temporomandibular joint region is conducted to obtain scanned image data; a three-dimensional solid mathematical model is generated based on the scanned image data, and the three-dimensional solid mathematical model comprises a temporomandibular joint region to be implanted; an implantation cutting line and a preset temporomandibular joint prosthesis are determined according to the three-dimensional solid mathematical model; and a temporomandibular joint prosthesis is implanted into the target temporomandibular joint region based on the implantation cutting line and the preset temporomandibular joint prosthesis. Compared with the prior art that the temporomandibular joint prosthesis implantation is carried out through the manual experience of operators, since according to the foregoing method of the present invention, the three-dimensional solid mathematical model is generated based on the scanned image data obtained by conducting total image scanning on the target temporomandibular joint region, and the implantation cutting line and the preset temporomandibular joint prosthesis that match the target temporomandibular joint region are determined according to the three-dimensional solid mathematical model, so that the temporomandibular joint prosthesis is implanted based on the implantation cutting line and the preset temporomandibular joint prosthesis, which avoids the technical problem in the prior art that the users still have poor temporomandibular joint movement after prosthesis implantation due to uncontrollable factors such as inexperience and incorrect operation of workers, and the like, which further ensures the implantation accuracy of the temporomandibular joint prosthesis.

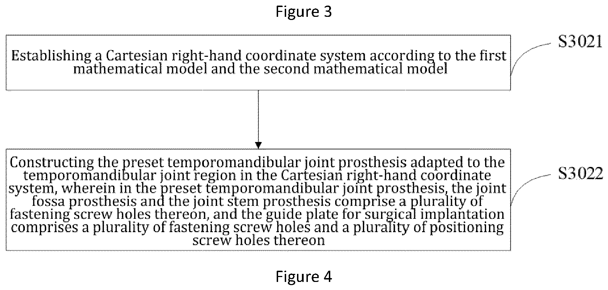

With reference to Figure 3, Figure 3 is a flow chart of a second embodiment of the temporomandibular joint prosthesis implantation method of the present invention.

Based on the foregoing first embodiment, in the present embodiment, in order to obtain the scanned image data in different states to improve the adaptability of the temporomandibular joint prosthesis implantation method in the present embodiment, the step S10 may comprise:
Step S101, adjusting an oral state corresponding to the target temporomandibular joint region to an occlusal state, and conducting total image scanning on the temporomandibular joint region in the occlusal state to obtain the first scanned image data.

Step S102, adjusting the oral state corresponding to the target temporomandibular joint region to a maximum mouth opening state, and conducting total image scanning on the temporomandibular joint region in the maximum mouth opening state to obtain the second scanned image data.

In a specific implementation, since the oral state corresponding to the target temporomandibular joint region is not always in a single state (for example, it is not always in a closed state or an open state), in the present embodiment, total image scanning on the temporomandibular joint region can be conducted in a minimum case (namely, the oral state corresponding to the target temporomandibular joint region is adjusted to the occlusal state) and a maximum case (namely, the oral state corresponding to the target temporomandibular joint region is adjusted to the maximum mouth opening state), so that the scanned image data can take into account various oral states, which improves the adaptability of the temporomandibular joint prosthesis implantation method in the present embodiment.

Further, in the present embodiment, in order to more accurately obtain the three-dimensional solid mathematical model in the occlusal state and the three-dimensional solid mathematical model in the maximum mouth opening state, the step S20 may comprise:
Step S201, conducting data processing on the scanned image data to obtain processed first scanned image data and processed second scanned image data.

In a specific implementation, appropriate methods can be selected for data processing according to the specific conditions of the scanned image data. For example, the scanned image data with poor visual quality can be processed by image enhancement (such as adjusting the image brightness, contrast and color saturation, etc.), the scanned image data with high noise can be processed by image filtering (such as filtering algorithm to eliminate noise, smooth the image or extract specific information from the image), and the scanned image data with insufficient integrity can be processed by image reconstruction (such as recovering high-quality images from damaged or incomplete image data), and the present embodiment does not limit the data processing method.

Step S202, generating the first mathematical model based on the processed first scanned image data.

It should be understood that the first mathematical model can represent a three-dimensional solid mathematical model when the oral state corresponding to the target temporomandibular joint region is adjusted to the occlusal state.

Step S203, generating the second mathematical model based on the processed second scanned image data.

It can be understood that the second mathematical model can represent a three-dimensional solid mathematical model when the oral state corresponding to the target temporomandibular joint region is adjusted to the maximum mouth opening state. Based on the foregoing first embodiment, in the present embodiment, in order to improve the adaptability of the preset temporomandibular joint prosthesis to the user, so as to improve the user's experience, the step S30 may comprise:
Step S301, locating the temporomandibular joint region to be implanted corresponding to the target temporomandibular joint region from the first mathematical model, and determining the implantation cutting line based on the temporomandibular joint region to be implanted, wherein the implantation cutting line comprises an upper cutting line and a lower cutting line.

Step S302, determining the preset temporomandibular joint prosthesis according to the first mathematical model and the second mathematical model, wherein the preset temporomandibular joint prosthesis comprises a joint fossa prosthesis, a joint head prosthesis, a joint stem prosthesis and a guide plate for surgical implantation.

It should be understood that the material of the joint fossa prosthesis can be ultrahigh molecular weight polyethylene, the material of the joint head prosthesis can be CoCrMo (Cobalt-Chrome-Molybdenum), and the material of the joint stem prosthesis can be TC4ELI (titanium alloy).

According to the present embodiment, adjusting an oral state corresponding to the target temporomandibular joint region to an occlusal state, and conducting total image scanning on the temporomandibular joint region in the occlusal state to obtain the first scanned image data; adjusting the oral state corresponding to the target temporomandibular joint region to a maximum mouth opening state, and conducting total image scanning on the temporomandibular joint region in the maximum mouth opening state to obtain the second scanned image data; conducting data processing on the scanned image data to obtain processed first scanned image data and processed second scanned image data; generating the first mathematical model based on the processed first scanned image data; generating the second mathematical model based on the processed second scanned image data; locating the temporomandibular joint region to be implanted corresponding to the target temporomandibular joint region from the first mathematical model, and determining the implantation cutting line based on the temporomandibular joint region to be implanted, wherein the implantation cutting line comprises an upper cutting line and a lower cutting line; and determining the preset temporomandibular joint prosthesis according to the first mathematical model and the second mathematical model, wherein the preset temporomandibular joint prosthesis comprises a joint fossa prosthesis, a joint head prosthesis, a joint stem prosthesis and a guide plate for surgical implantation. Compared with the traditional temporomandibular joint prosthesis implantation methods, the foregoing method in the present embodiment generates the corresponding three-dimensional solid mathematical models based on the scanned image data in different oral states, which improves the implantation adaptability of temporomandibular joint prosthesis.

With reference to Figure 4, Figure 4 is a flow chart of a third embodiment of the temporomandibular joint prosthesis implantation method of the present invention.

Based on the foregoing each embodiment, in the present embodiment, in order to further improve the adaptability of the preset temporomandibular joint prosthesis to the user, so as to further improve the user's experience, the step S302 may comprise: Step S3021, establishing a Cartesian right-hand coordinate system according to the first mathematical model and the second mathematical model.

It should be noted that the Cartesian right-hand coordinate system can be a three-dimensional coordinate system, which adopts three mutually perpendicular axes to describe the position and direction in space, namely, X-axis, Y-axis and Z-axis.

In a specific implementation, the Cartesian right-hand coordinate system can be established by taking the model center point of the first mathematical model and the second mathematical model as the origin.

Step S3022, constructing the preset temporomandibular joint prosthesis adapted to the temporomandibular joint region in the Cartesian right-hand coordinate system, wherein in the preset temporomandibular joint prosthesis, the joint fossa prosthesis and the joint stem prosthesis comprise a plurality of fastening screw holes thereon, and the guide plate for surgical implantation comprises a plurality of fastening screw holes and a plurality of positioning screw holes thereon.

Based on the foregoing each embodiment, in the present embodiment, in order to ensure the implantation stability of the preset temporomandibular joint prosthesis, the step S40 may comprise:
Step S401, cutting the temporomandibular joint region to be implanted based on the implantation cutting line, and implanting the preset temporomandibular joint prosthesis into the target temporomandibular joint region by using screws with the fastening screw holes as the fastening reference and the positioning screw holes as the positioning reference after completing cutting.

Step S50, when the preset temporomandibular joint prosthesis is implanted into the target temporomandibular joint region, removing the guide plate for surgical implantation and stitching the wound surface corresponding to the target temporomandibular joint region.

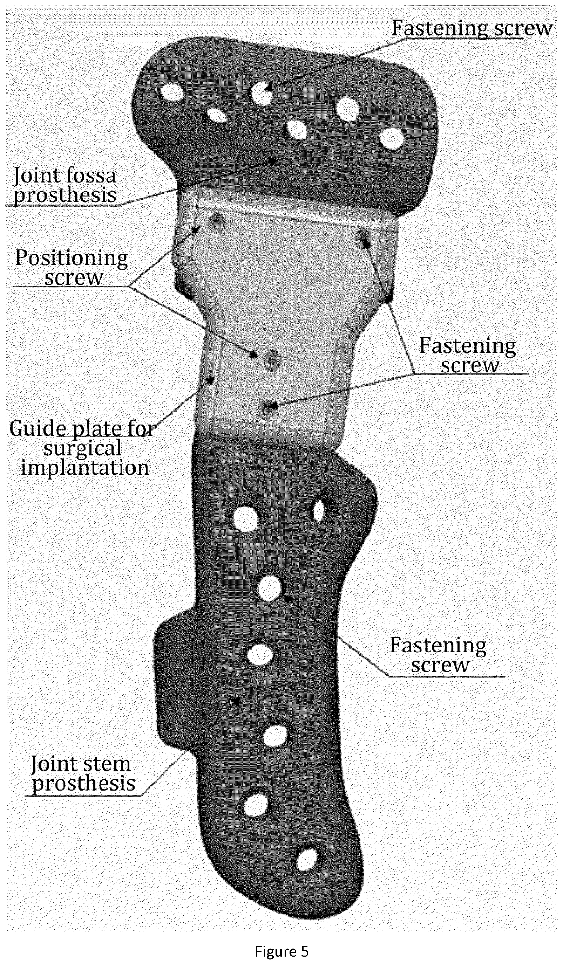

In a specific implementation, the guide plate for surgical implantation is taken as an auxiliary tool in the present embodiment, so when the preset temporomandibular joint prosthesis has been implanted into the target temporomandibular joint region, the guide plate for surgical implantation can be removed. With reference to Figure 5, Figure 5 is a schematic diagram of a preset temporomandibular joint prosthesis in the temporomandibular joint prosthesis implantation method of the present invention. In Figure 5, the joint fossa prosthesis and the joint stem prosthesis comprise a plurality of fastening screw holes thereon, and the guide plate for surgical implantation comprises a plurality of fastening screw holes and a plurality of positioning screw holes thereon. The joint head prosthesis is arranged at a junction of the joint fossa prosthesis and the joint stem prosthesis, but it is not marked in the figure because it is blocked by the guide plate for surgical implantation. Particularly, the material of the positioning screws and the fastening screws can also be TC4ELI (titanium alloy).

According to the present embodiment, establishing a Cartesian right-hand coordinate system according to the first mathematical model and the second mathematical model; constructing the preset temporomandibular joint prosthesis adapted to the temporomandibular joint region in the Cartesian right-hand coordinate system, wherein in the preset temporomandibular joint prosthesis, the joint fossa prosthesis and the joint stem prosthesis comprise a plurality of fastening screw holes thereon, and the guide plate for surgical implantation comprises a plurality of fastening screw holes and a plurality of positioning screw holes thereon; cutting the temporomandibular joint region to be implanted based on the implantation cutting line, and implanting the preset temporomandibular joint prosthesis into the target temporomandibular joint region by using screws with the fastening screw holes as the fastening reference and the positioning screw holes as the positioning reference after completing cutting; and when the preset temporomandibular joint prosthesis is implanted into the target temporomandibular joint region, removing the guide plate for surgical implantation and stitching the wound surface corresponding to the target temporomandibular joint region. Compared with the traditional temporomandibular joint prosthesis implantation method, the foregoing method of the present embodiment implants the preset temporomandibular joint prosthesis into the target temporomandibular joint region by using screws with the fastening screw holes as the fastening reference and the positioning screw holes as the positioning reference after completing cutting, which improves the implantation accuracy and stability of the temporomandibular joint prosthesis in the present embodiment.

In addition, the embodiment of the present invention further provides a storage medium, wherein the temporomandibular joint prosthesis implantation program is stored on the storage medium, and when the temporomandibular joint prosthesis implantation program is executed by the processor, the steps of the temporomandibular joint prosthesis implantation method described above are carried out.

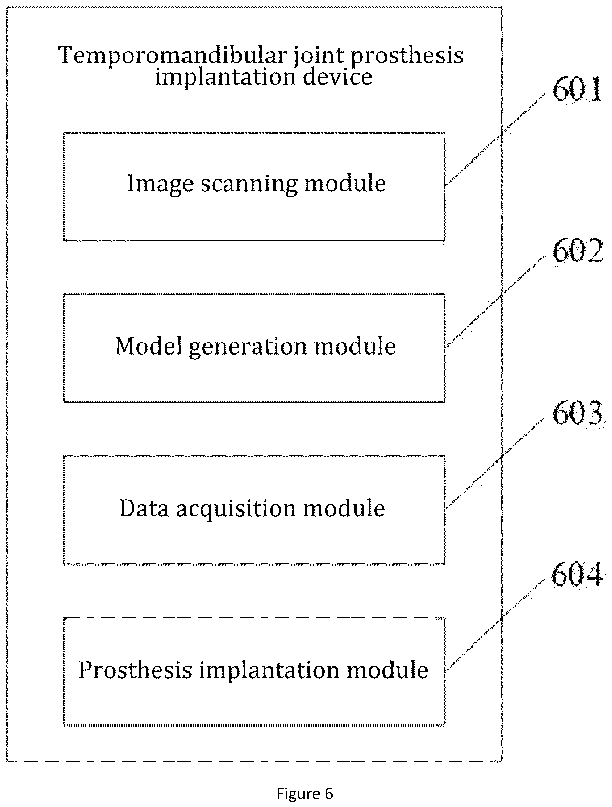

With reference to Figure 6, Figure 6 is a structural block diagram of a first embodiment of a temporomandibular joint prosthesis implantation device of the present invention. As shown in Figure 6, a temporomandibular joint prosthesis implantation device proposed by the embodiment of the present invention comprises:
An image scanning module 601, configured to conduct total image scanning on a target temporomandibular joint region to obtain scanned image data;
A model generation module 602, configured to generate a three-dimensional solid mathematical model based on the scanned image data, wherein the three-dimensional solid mathematical model comprises a temporomandibular joint region to be implanted;
A data acquisition module 603, configured to determine an implantation cutting line and a preset temporomandibular joint prosthesis according to the three-dimensional solid mathematical model;
A prosthesis implantation module 604, configured to implant a temporomandibular joint prosthesis in the target temporomandibular joint region based on the implantation cutting line and the preset temporomandibular joint prosthesis.

According to the present embodiment, total image scanning on a target temporomandibular joint region is conducted to obtain scanned image data; a three-dimensional solid mathematical model is generated based on the scanned image data, and the three-dimensional solid mathematical model comprises a temporomandibular joint region to be implanted; an implantation cutting line and a preset temporomandibular joint prosthesis are determined according to the three-dimensional solid mathematical model; and a temporomandibular joint prosthesis is implanted into the target temporomandibular joint region based on the implantation cutting line and the preset temporomandibular joint prosthesis. Compared with the prior art that the temporomandibular joint prosthesis implantation is realized through the manual experience of workers, since according to the foregoing method of the present invention, the three-dimensional solid mathematical model is generated based on the scanned image data obtained by conducting total image scanning on the target temporomandibular joint region, and the implantation cutting line and the preset temporomandibular joint prosthesis that match the target temporomandibular joint region are determined according to the three-dimensional solid mathematical model, so that the temporomandibular joint prosthesis is implanted based on the implantation cutting line and the preset temporomandibular joint prosthesis, which avoids the technical problem in the prior art that the users still have poor temporomandibular joint movement after prosthesis implantation due to uncontrollable factors such as inexperience and incorrect operation of workers, and the like, which further ensures the implantation accuracy of the temporomandibular joint prosthesis.

Based on the first embodiment of the temporomandibular joint prosthesis implantation device of the present invention, a second embodiment of the temporomandibular joint prosthesis implantation device of the present invention is proposed.

In the embodiment, the image scanning module 601 is further configured to adjust an oral state corresponding to the target temporomandibular joint region to an occlusal state, and conduct total image scanning on the temporomandibular joint region in the occlusal state to obtain the first scanned image data; and to adjust the oral state corresponding to the target temporomandibular joint region to a maximum mouth opening state, and conduct total image scanning on the temporomandibular joint region in the maximum mouth opening state to obtain the second scanned image data. Further, the model generation module 602, is further configured to conduct data processing on the scanned image data to obtain processed first scanned image data and processed second scanned image data; and to generate the first mathematical model based on the processed first scanned image data; and to generate the second mathematical model based on the processed second scanned image data.

Further, the data acquisition module 603, is further configured to locate the temporomandibular joint region to be implanted corresponding to the target temporomandibular joint region from the first mathematical model, and determine the implantation cutting line based on the temporomandibular joint region to be implanted, wherein the implantation cutting line comprises an upper cutting line and a lower cutting line; and to determine the preset temporomandibular joint prosthesis according to the first mathematical model and the second mathematical model, wherein the preset temporomandibular joint prosthesis comprises a joint fossa prosthesis, a joint head prosthesis, a joint stem prosthesis and a guide plate for surgical implantation.

Further, the data acquisition module 603, is further configured to establish a Cartesian right-hand coordinate system according to the first mathematical model and the second mathematical model; and to construct the preset temporomandibular joint prosthesis adapted to the temporomandibular joint region in the Cartesian right-hand coordinate system, wherein in the preset temporomandibular joint prosthesis, the joint fossa prosthesis and the joint stem prosthesis comprise a plurality of fastening screw holes thereon, and the guide plate for surgical implantation comprises a plurality of fastening screw holes and a plurality of positioning screw holes thereon.

Further, the prosthesis implantation module 604, is further configured to cut the temporomandibular joint region to be implanted based on the implantation cutting line, and implant the preset temporomandibular joint prosthesis into the target temporomandibular joint region by using screws with the fastening screw holes as the fastening reference and the positioning screw holes as the positioning reference after completing cutting.

Further, the prosthesis implantation module 604, is further configured to remove the guide plate for surgical implantation and stitching the wound surface corresponding to the target temporomandibular joint region when the preset temporomandibular joint prosthesis is implanted into the target temporomandibular joint region.

Other embodiments or specific implementations of the temporomandibular joint prosthesis implantation device of the present invention can refer to each of the foregoing method embodiments, which will not be repeated herein.

It should be noted that in the text, the terms "comprise", "contain" or any other variation thereof are intended to cover non-exclusive inclusion, so that a process, method, article or system including a series of elements includes not only those elements, but also other elements not explicitly listed, or elements inherent to such process, method, article or system. Without more restrictions, an element defined by the phrase "comprising one..." does not exclude the existence of other identical elements in the process, method, article or system that includes the element.

The serial numbers of the foregoing embodiments of the present invention are only for description and do not represent the advantages and disadvantages of the embodiments.

Through the description of the foregoing embodiments, those skilled in the art can clearly understand that the methods of the foregoing embodiments can be realized by means of software and necessary general hardware platform, and they can also definitely be realized by hardware, but in many cases, the former is the better embodiment. Based on the understanding, the technical solutions of the present invention essentially or the part that contributes to the prior art can be embodied in a form of a software product, and the software product is stored in a storage medium (such as a read-only memory/a random access memory, a magnetic disk and an optical disk) and comprises several instructions to make a terminal device (which can be a mobile phone, a computer, a server or a network device, etc.) execute the methods described in each of the embodiments of the present invention.

The foregoing are only preferred embodiments of the present invention, which does not limit the patent scope of the present invention. Any equivalent structure or equivalent process transformation made by using the description and attached drawings of the present invention, or directly or indirectly applied to other related technical fields, shall equally fall within the patent protection scope of the present invention.

## Claims

1. A temporomandibular joint prosthesis implantation device, comprising:
an image scanning module, configured to conduct total image scanning on a target temporomandibular joint region to obtain scanned image data;
a model generation module, configured to generate a three-dimensional solid mathematical model based on the scanned image data, wherein the three-dimensional solid mathematical model comprises a temporomandibular joint region to be implanted;
a data acquisition module, configured to determine an implantation cutting line and a preset temporomandibular joint prosthesis according to the three-dimensional solid mathematical model;
a prosthesis implantation module, configured to implant a temporomandibular joint prosthesis in the target temporomandibular joint region based on the implantation cutting line and the preset temporomandibular joint prosthesis;
wherein, the scanned image data comprises first scanned image data and second scanned image data, and the step of conducting total image scanning on the target temporomandibular joint region to obtain the scanned image data comprises:
adjusting an oral state corresponding to the target temporomandibular joint region to an occlusal state, and conducting total image scanning on the temporomandibular joint region in the occlusal state to obtain the first scanned image data;
adjusting the oral state corresponding to the target temporomandibular joint region to a maximum mouth opening state, and conducting total image scanning on the temporomandibular joint region in the maximum mouth opening state to obtain the second scanned image data;
the three-dimensional solid mathematical model comprises a first mathematical model and a second mathematical model, wherein the step of generating the three-dimensional solid mathematical model based on the scanned image data comprises:
conducting data processing on the scanned image data to obtain processed first scanned image data and processed second scanned image data;
generating the first mathematical model based on the processed first scanned image data;
generating the second mathematical model based on the processed second scanned image data;
wherein the step of determining the implantation cutting line and the preset temporomandibular joint prosthesis according to the three-dimensional solid mathematical model comprises:
locating the temporomandibular joint region to be implanted corresponding to the target temporomandibular joint region from the first mathematical model,
and determining the implantation cutting line based on the temporomandibular joint region to be implanted, wherein the implantation cutting line comprises an upper cutting line and a lower cutting line;
determining the preset temporomandibular joint prosthesis according to the first mathematical model and the second mathematical model, wherein the preset temporomandibular joint prosthesis comprises a joint fossa prosthesis, a joint head prosthesis, a joint stem prosthesis and a guide plate for surgical implantation;
wherein the step of determining the preset temporomandibular joint prosthesis according to the first mathematical model and the second mathematical model comprises:
establishing a Cartesian right-hand coordinate system according to the first mathematical model and the second mathematical model; and
constructing the preset temporomandibular joint prosthesis adapted to the temporomandibular joint region in the Cartesian right-hand coordinate system,
wherein in the preset temporomandibular joint prosthesis, the joint fossa prosthesis and the joint stem prosthesis comprise a plurality of fastening screw holes thereon, and the guide plate for surgical implantation comprises a plurality of fastening screw holes and a plurality of positioning screw holes thereon.

2. Temporomandibular joint prosthesis implantation equipment, comprising a memory, a processor, and a temporomandibular joint prosthesis implantation program that is stored in the memory and can run on the processor, wherein the temporomandibular joint prosthesis implantation program is configured to carry out any step as claimed in claim 1.

3. A storage medium, wherein a temporomandibular joint prosthesis implantation program is stored on the storage medium, and when the temporomandibular joint prosthesis implantation program is executed by a processor, any step of claim 1 is carried out.
